# FASCICULE DE BREVET EUROPEEN

(11) **EP 1 489 980 B1**
(45) Date de publication et mention de la délivrance du brevet: **23.04.2008**
(21) Numéro de dépôt: 03745810.6
(22) Date de dépôt: 02.04.2003
(51) Int. Cl.: A61B 17/70

(54) **SYSTEME D'OSTEOSYNTHESE RACHIDIENNE**
WIRBELSÄULEN-OSTEOSYNTHESESYSTEM
SPINAL OSTEOSYNTHESIS SYSTEM

(30) Priorité: 04.04.2002 FR 0204181
(43) Date de publication de la demande: 29.12.2004
(73) Titulaire: KISCOMEDICA, 69800 Saint-Priest (FR)
(72) Inventeur: RAMARE, Stéphane, F-78150 LE CHESNAY (FR)
(74) Mandataire: Flavenot, Bernard
(86) Numéro de dépôt international: PCT/FR2003/001017
(87) Numéro de publication internationale: WO 2003/084415

(56) Documents cités:
- EP-A- 1 023 873
- DE-A- 3 841 008
- FR-A- 2 780 631
- US-A- 5 569 247

## Description

La présente invention concerne les systèmes d'ostéosynthèse rachidienne qui trouvent une application particulièrement avantageuse pour le maintien de deux vertèbres, consécutives ou non, l'une par rapport à l'autre en vue de la réalisation d'une arthrodèse rachidienne, par exemple chez un être humain dans le but de supprimer par exemple la cause des douleurs générées par une fracture de vertèbre ou pour éviter le risque de complication paralytique liée à cette fracture.

Les praticiens dans le domaine de la chirurgie rachidienne utilisent notamment des systèmes comportant essentiellement une plaque dans laquelle sont pratiqués un orifice et une lumière, et des vis comportant chacune une tige à filetage osseux terminée par une tête d'épaulement, ces vis étant aptes à coopérer avec la plaque pour que la plaque en appui contre les vertèbres soit emprisonnée entre ces vertèbres et les têtes d'épaulement des vis.

Ces systèmes d'ostéosynthèse rachidienne présentent des inconvénients car, notamment, les épiphyses des vertèbres rendent difficile la mise en place des vis à filetage osseux dans les orifices des plaques pour leur vissage dans les corps vertébraux, et ne permettent pas au praticien d'ajuster facilement la distance séparant les deux segments de vertèbres pour éliminer le foyer de la douleur et rétablir la hauteur normale du segment de colonne vertébrale.

Pour tenter de pallier ces inconvénients, il a été mis au point des systèmes comportant des vis pédiculaires, des pattes, des premiers moyens pour fixer une première extrémité des pattes sur les têtes des vis pédiculaires, au moins une plaque ou une tige et des seconds moyens pour fixer la plaque ou la tige sur la seconde extrémité des pattes. Par exemple, le document EP-1023873-A2 décrit un système d'ostéosynthèse rachidienne comportant au moins une vis pédiculaire comportant une tête, une patte, des premiers moyens pour fixer une première extrémité de la patte sur la tête de la vis pédiculaire, une tige et des seconds moyens pour fixer la tige sur la seconde extrémité de la patte. Les premiers moyens pour fixer la première extrémité de la patte sur la tête de la vis pédiculaire comportent une première partie de forme hémisphérique solidaire de la tête de la vis pédiculaire, une deuxième partie de forme hémisphérique solidaire de la première extrémité de la patte, cette deuxième partie hémisphérique étant complémentaire de la première partie hémisphérique, et des moyens de solidarisation pour fixer entre elles les première et deuxième parties hémisphériques quand elles coopèrent l'une dans l'autre en ayant le même premier centre de courbure. Ces systèmes sont cependant encore relativement complexes aussi bien pour leur mise en place que pour l'ajustement optimal des différentes distances.

La présente invention a donc pour but de réaliser un système d'ostéosynthèse rachidienne qui permet de pallier en grande partie les inconvénients des systèmes de l'art antérieur mentionnés ci-dessus

Plus précisément, la présente invention a pour objet un système d'ostéosynthèse rachidienne comportant au moins une vis pédiculaire comprenant une tête, une patte, des premiers moyens pour fixer une première extrémité de la patte sur la tête de la vis pédiculaire, une plaque et des seconds moyens pour fixer la plaque sur la seconde extrémité de la patte, caractérisé par le fait que les premiers moyens pour fixer la première extrémité de la patte sur la tête de la vis pédiculaire comportent :
une première partie de forme hémisphérique solidaire de la tête de la vis pédiculaire,
une deuxième partie de forme hémisphérique solidaire de la première extrémité de la patte, cette deuxième partie hémisphérique étant complémentaire de la première partie hémisphérique, et
des moyens de solidarisation pour fixer entre elles les première et deuxième parties hémisphériques quand elles coopèrent l'une dans l'autre en ayant le même premier centre de courbure,
et que les seconds moyens pour fixer la plaque sur la seconde extrémité de la patte comportent :
une troisième partie de forme hémisphérique solidaire de la plaque,
une quatrième partie de forme hémisphérique solidaire de la seconde extrémité de la patte, cette quatrième partie hémisphérique étant complémentaire de la troisième partie hémisphérique, et
des moyens de solidarisation pour fixer entre elles les troisième et quatrième parties hémisphériques quand elles coopèrent l'une dans l'autre en ayant le même second centre de courbure.

D'autres caractéristiques et avantages de l'invention apparaîtront au cours de la description suivante donnée en regard des dessins annexés à titre illustratif mais nullement limitatif, dans lesquels :
La figure 1 représente une vue en coupe transversale d'une partie d'un mode de réalisation du système d'ostéosynthèse rachidienne selon l'invention, cette coupe étant définie dans le plan P référencé sur la figure 2, et
La figure 2 représente une vue en perspective d'un mode de réalisation du système d'ostéosynthèse rachidienne en accord avec la représentation selon la figure 1.

Il est bien précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

La Demanderesse tient aussi à préciser que les figures ne représentent qu'un mode de réalisation de l'objet selon l'invention, mais qu'il peut exister d'autres modes de réalisation qui répondent à la définition de cette invention.

Elle précise en outre que, lorsque, selon la définition de l'invention, l'objet de l'invention comporte "au moins un" élément ayant une fonction donnée, le mode de réalisation décrit peut comporter plusieurs de ces éléments.

Elle précise aussi que, si le mode de réalisation de l'objet selon l'invention tel qu'illustré comportent plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

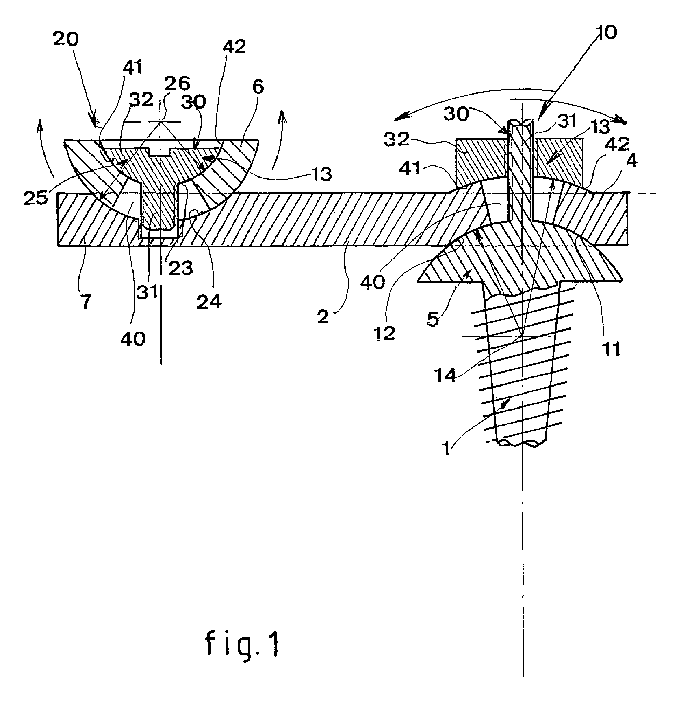

Par référence à la figure 1, le système d'ostéosynthèse rachidienne comporte au moins une vis pédiculaire 1 comprenant une tête 5, une patte 2, des premiers moyens 10 pour fixer une première extrémité 4 de la patte 2 sur la tête 5 de la vis pédiculaire, une plaque 6 et des seconds moyens 20 pour fixer la plaque 6 sur la seconde extrémité 7 de la patte 2.

Selon une caractéristique de l'invention, les premiers moyens 10 pour fixer la première extrémité 4 de la patte 2 sur la tête 5 de la vis pédiculaire comportent une première partie 11 de formes hémisphérique solidaire de la tête 5 de la vis pédiculaire 1, une deuxième partie 12 de forme hémisphérique solidaire de la première extrémité 4 de la patte 2, cette deuxième partie hémisphérique 12 étant complémentaire de la première partie hémisphérique 11, et des moyens de solidarisation 13 pour fixer entre elles les première et deuxième parties hémisphériques 11, 12 quand elles coopèrent l'une dans l'autre en ayant le même premier centre de courbure 14.

Quant aux seconds moyens 20 pour fixer la plaque 6 sur la seconde extrémité 7 de la patte 2, ils comportent une troisième partie 23 de forme hémisphérique solidaire de la plaque 6, une quatrième partie 24 de forme hémisphérique solidaire de la seconde extrémité 7 de la patte 2, cette quatrième partie hémisphérique 24 étant complémentaire de la troisième partie hémisphérique 23, et des moyens de solidarisation 25 pour fixer entre elles les troisième et quatrième parties hémisphériques 23, 24 quand elles coopèrent l'une dans l'autre en ayant le même second centre de courbure 26.

Dans une réalisation particulièrement avantageuse, les moyens de solidarisation 13 et/ou 25 définis ci-dessus comportent une vis de fixation 30 constituée d'une tige filetée 31 et d'une tête de vissage 32, et des moyens pour monter la vis de fixation 30 en coopération avec les deux parties hémisphériques 11-12 et/ou 23-24 de façon à prendre l'une de ces deux parties en sandwich entre l'autre partie et la tête de vissage 32.

De façon préférentielle, les moyens pour monter la vis de fixation 30 en coopération avec les deux parties hémisphériques pour prendre l'une de ces deux parties en sandwich entre l'autre partie et la tête de vissage, sont constitués, comme les moyens 13 représentés sur la partie droite de la figure 1, par le fait que la tige filetée 31 est solidaire de la partie hémisphérique prenant l'autre en sandwich et traverse cette autre partie hémisphérique par une percée 40 d'une section supérieure à celle de la tige filetée 31, la tête de vissage 32 se vissant alors sur la tige filetée 31, les deux faces 41-42 de la tête de vissage 32 et de la partie hémisphérique venant au contact l'une de l'autre étant de formes hémisphériques complémentaires et ayant des centres de courbure sensiblement confondus avec le centre de courbure 14 des parties hémisphériques coopérant l'une dans l'autre.

Avantageusement, lorsque les première et seconde parties hémisphériques 11, 12 sont respectivement convexe et concave, les deux faces 41, 42 de la tête de vissage 32 et de la partie hémisphérique venant au contact l'une de l'autre sont respectivement concave et convexe, comme illustré sur cette partie droite de la figure 1.

Cependant, selon une autre de mode de réalisation, les moyens pour monter la vis de fixation 30 en coopération avec les deux parties hémisphériques pour prendre l'une de ces deux parties en sandwich entre l'autre partie et la tête de vissage 32, sont constitués, comme les moyens 13 représentés sur la partie gauche de la figure 1, par le fait que la tige filetée 31 se visse dans la partie hémisphérique prenant l'autre en sandwich et traverse cette autre partie par une percée 40 d'une section supérieure à celle de la tige filetée 31, la tête de vissage 32 étant solidaire de la tige filetée 31, les deux faces 41, 42 de la tête de vissage 32 et de la partie hémisphérique venant au contact étant de formes hémisphériques complémentaires et ayant des centres de courbure sensiblement confondus avec le centre de courbure 26 des parties hémisphériques coopérant l'une dans l'autre.

Avantageusement, lorsque les troisième et quatrième parties hémisphériques 23, 24 sont respectivement convexe et concave, les deux faces 41, 42 de la tête de vissage 32 et de la partie hémisphérique venant au contact sont respectivement convexe et concave, comme illustré sur cette partie gauche de la figure 1.

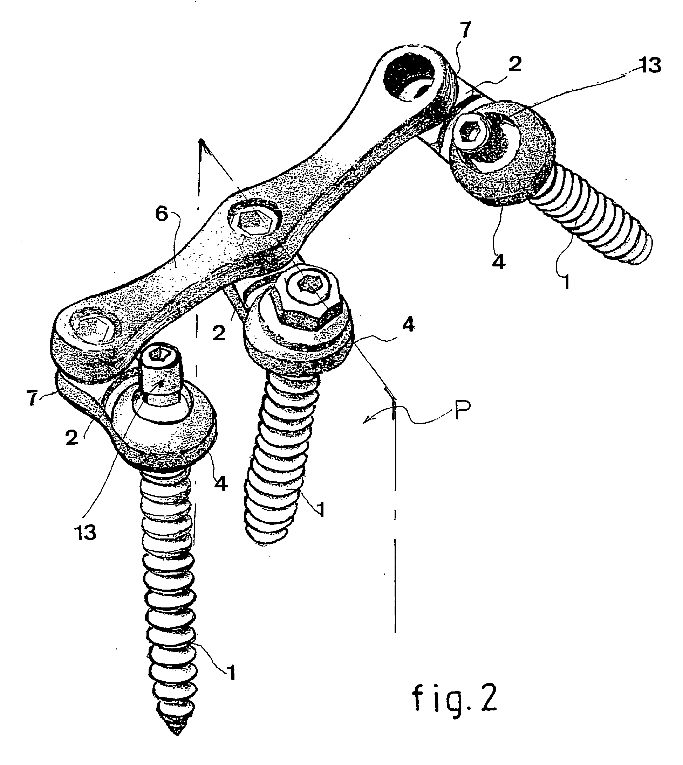

Le mode de réalisation du systèmes d'ostéosynthèse rachidienne selon l'invention décrit ci-dessus comporte, pour sa définition essentielle, au moins une vis pédiculaire 1, une patte 2 par vis pédiculaire et au moins une plaque 6. Il est cependant bien évident que, dans la pratique, un tel système sera par exemple réalisé comme celui qui est illustré sur la figure 2.

Dans le mode de réalisation selon la figure 2, le système comporte plusieurs vis pédiculaires 1, au nombre de trois sur cette figure 2, une patte 2 par vis pédiculaire et une plaque 6, cette plaque 6 comportant au moins autant de troisièmes parties hémisphériques 23 que dé pattes 2 et donc de vis pédiculaires.

Cependant, dans d'autres modes de réalisation possibles, le système peut comporter plusieurs pattes par vis pédiculaire, les premières extrémités de ces pattes se superposant les unes sur les autres pour obtenir une rotation des unes par rapport aux autres.

Pour implanter un système d'ostéosynthèse rachidienne comme celui illustré sur la figure 2, le praticien commence par visser les trois vis pédiculaires 1 dans les pédicules des vertèbres. Etant donnée la difficulté pour implanter de telles vis, il est impossible d'obtenir que celles-ci soient parfaitement parallèles les unes par rapport aux autres et que leurs têtes soient parfaitement alignées sur une même droite. Dans ces conditions, il est pratiquement impossible de positionner sur les têtes de ces trois vis une plaque qui est généralement rectiligne.

Avec le système d'ostéosynthèse rachidienne selon l'invention, après qu'il ait vissé à fond les vis pédiculaires dans les pédicules, le praticien positionne une patte 2 sur chaque vis pédiculaire de façon que les première et deuxième parties hémisphériques 11, 12 se superposent comme illustré sur les figures 1 et 2, la tige filetée 31 passant à travers la percée 40, la tête de vissage 32 étant partiellement vissée.

Le praticien monte ensuite en coopération la plaque 6 avec les secondes extrémités 7 des pattes 2 de façon que les troisième et quatrième parties hémisphériques 23, 24 coopèrent l'une dans l'autre, puis visse partiellement les tiges filetées 31 dans les pattes 2 (partie gauche de la figure 1).

Avec un ancillaire bien connu en lui-même, le praticien distracte les vertèbres pour qu'elles prennent leur position relative souhaitée. Dans ce mouvement relatif des vertèbres les unes par rapport aux autres, les pattes 2 et la plaque 6 se déplacent les unes par rapport aux autres et, grâce aux parties hémisphériques 11-12, 23-24, trouvent toujours la position optimale voulue.

Le praticien bloque alors les parties hémisphériques au moyen des vis de fixation 30, et peut enlever l'ancillaire de distraction.

Avec le système d'ostéosynthèse rachidienne selon l'invention dont les caractéristiques structurelles sont définies ci-dessus, il est donc très facilement possible de positionner la plaque 6 par rapport aux vis pédiculaires 1, sachant que chaque patte 2 peut se déplacer dans un cône solide autour de la tête 5 de la vis pédiculaire qui lui est associée, et que la plaque 6 peut se déplacer dans un cône solide autour de la seconde extrémité 7 de cette patte 2.

A la description ci-dessus, apparaissent les principaux avantages du système d'ostéosynthèse rachidienne selon l'invention. A savoir : les vis pédiculaires 1 peuvent être implantées relativement indépendamment les unes par rapport aux autres, la plaque 6 peut toujours être reliée aux vis pédiculaires quelle que soit la position relative de ces dernières et, étant positionnée latéralement par rapport aux vis pédiculaires, sa mise en place n'est pas gênée par les épiphyses épineuses.

## Revendications

1. Système d'ostéosynthèse rachidienne comportant au moins une vis pédiculaire (1) comportant une tête (5), une patte (2), des premiers moyens (10) pour fixer une première extrémité (4) de la patte (2) sur la tête (5) de la vis pédiculaire, une plaque (6) et des seconds moyens (20) pour fixer la plaque (6) sur la seconde extrémité (7) de la patte (2), les premiers moyens (10) pour fixer la première extrémité (4) de la patte sur la tête (5) de la vis pédiculaire comportant :
une première partie (11) de forme hémisphérique solidaire de la tête (5) de la vis pédiculaire (1),
une deuxième partie (12) de forme hémisphérique solidaire de la première extrémité (4) de la patte (2), cette deuxième partie hémisphérique (12) étant complémentaire de la première partie hémisphérique (11), et
des premiers moyens de solidarisation (13) pour fixer entre elles les première et deuxième parties hémisphériques (11, 12) quand elles coopèrent l'une dans l'autre en ayant le même premier centre de courbure (14),
et les seconds moyens (20) pour fixer la plaque (6) sur la seconde extrémité (7) de la patte (2), comportant :
une troisième partie (23) de forme hémisphérique solidaire de la plaque (6),
une quatrième partie (24) de forme hémisphérique solidaire de la seconde extrémité (7) de la patte (2), cette quatrième partie hémisphérique (24) étant complémentaire de la troisième partie hémisphérique (23), et
des seconds moyens de solidarisation (25) pour fixer entre elles les troisième et quatrième parties hémisphériques (23, 24) quand elles coopèrent l'une dans l'autre en ayant le même second centre de courbure (26).

2. Système selon la revendication 1, **caractérisé par le fait que** les premier et second moyens de solidarisation (13, 25) comportent une vis de fixation (30) constituée d'une tige filetée (31) et d'une tête de vissage (32), des moyens pour monter ladite vis de fixation (30) en coopération avec les deux parties hémisphériques (11-12, 23-24) de façon à prendre l'une de ces deux parties en sandwich entre l'autre partie et la tête de vissage (32).

3. Système selon la revendication 2, **caractérisé par le fait que** les moyens pour monter ladite vis de fixation (30) en coopération avec les deux parties hémisphériques pour prendre l'une de ces deux parties en sandwich entre l'autre partie et la tête de vissage (32), sont constitués **par le fait que** la tige filetée (31) est solidaire de la partie hémisphérique prenant l'autre en sandwich et traverse cette autre partie hémisphérique par une percée (40) d'une section supérieure à celle de ladite tige filetée (31), la tête de vissage (32) se vissant sur ladite tige filetée (31), les deux faces (41, 42) de la tête de vissage (32) et de la partie hémisphérique venant au contact l'une de l'autre étant de formes hémisphériques complémentaires et ayant des centres de courbure sensiblement confondus avec le centre de courbure (14, 26) des parties hémisphériques coopérant l'une dans l'autre.

4. Système selon la revendication 3, **caractérisé par le fait que**, les première et seconde parties hémisphériques (11, 12) étant respectivement convexe et concave, les deux faces (41, 42) de la tête de vissage (32) et de la partie hémisphérique venant au contact l'une de l'autre sont respectivement concave et convexe.

5. Système selon la revendication 2, **caractérisé par le fait que** les moyens pour monter ladite vis de fixation (30) en coopération avec les deux parties hémisphériques pour prendre l'une de ces deux parties en sandwich entre l'autre partie et la tête de vissage (32), sont constitués **par le fait que** la tige filetée (31) se visse dans la partie hémisphérique prenant l'autre en sandwich et traverse cette autre partie par une percée (40) d'une section supérieure à celle de ladite tige filetée (31), la tête de vissage (32) étant solidaire de ladite tige filetée (31), les deux faces (41, 42) de la tête de vissage (32) et de la partie hémisphérique venant au contact l'une de l'autre étant de formes hémisphériques complémentaires et ayant des centres de courbure sensiblement confondus avec le centre de courbure (14, 26) des parties hémisphériques coopérant l'une dans l'autre.

6. Système selon la revendication 5, **caractérisé par le fait que**, les troisième et quatrième parties hémisphériques (23, 24) étant respectivement convexe et concave, les deux faces (41, 42) de la tête de vissage (32) et de la partie hémisphérique venant au contact l'une de l'autre sont respectivement convexe et concave.

## Claims

1. A spinal osteosynthesis system comprising at least one pedicular screw (1) with a head (5), a lug (2), first means (10) for fastening a first end (4) of the lug (2) onto the head (5) of the pedicular screw, a plate (6) and second means (20) for fixing the plate (6) onto the second end (7) of the lug (2), the first means (10) for fastening the first end (4) of the lug onto the pedicular screw head (5) comprising:
a first portion (11) of hemispherical shape secured to the head (5) of the pedicular screw (1);
- a second portion (12) of hemispherical shape secured to the first end (4) of the lug (2), this second hemispherical portion (12) being complementary to the first hemispherical portion (11); and
- first fastener means (13) for fastening together the first and second hemispherical portions (11, 12) when they cooperate one in the other with a common first center of curvature (14), and
the second means (20) for fastening the plate (6) onto the second end (7) of the lug (2) comprising:
- a third portion (23) of hemispherical shape secured to the plate (6),
- a fourth portion (24) of hemispherical shape secured to the second end (7) of the lug (2), this fourth hemispherical portion (24) being complementary to the third hemispherical portion (23), and
- second fastener means (25) for fastening together the third and fourth hemispherical portions (23, 24) when they cooperate one in the other with a common second center of curvature (26).

2. A system according to claim 1, **characterized by** the fact that the first and second fastener means (13, 25) comprise a fastening screw (30) made up of a threaded shank (31) and a screwing head (32), and means for assembling said fastening screw (30) in cooperation with the two hemispherical portions (11-12, 23-24) so as to sandwich one of the two portions between the other portion and the screwing head (32).

3. A system according to claim 2, **characterized by** the fact that the means for assembling said fastening screw (30) in cooperation with the two hemispherical portions for sandwiching one of the two portions between the other portion and the screwing head (32), are constituted by the fact that the threaded shank (31) is secured to the hemispherical portion sandwiching the other hemispherical portion and goes through it via a hole (40) of section greater than that of the said threaded shank (31), the screwing head (32) being screwed onto said threaded shank (31), the two faces (41, 42) of the screwing head (32) and of the hemispherical portion that come into contact with each other being of complementary hemispherical shapes and with centers of curvature that coincide substantially with the centers of curvature (14,26) of the hemispherical portions cooperating one in the other.

4. A system according to claim 3, **characterized by** the fact that the first and second hemispherical portions (11, 12) being respectively convex and concave, the two faces (41, 42) of the screwing head (32) and of the hemispherical portion that come into contact with each other are respectively concave and convex.

5. A system according to claim 2, **characterized by** the fact that the means for assembling said fastening screw (30) in cooperation with the two hemispherical portions for sandwiching one of the two portions between the other portion and the screwing head (32) are constituted by the fact that the threaded shank (31) is screwed into the hemispherical portion sandwiching the other portion and goes through this other portion via a hole (40) of section greater than that of the said threaded shank (31), the screwing head (32) being secured to the threaded shank (31), the two faces (41, 42) of the screwing head (32) and of the hemispherical portion that come into contact with each other being of complementary hemispherical shapes and with centers of curvature that coincide substantially with the centers of curvature (14, 26) of the hemispherical portions cooperating one in the other.

6. A system according to claim 5, **characterized by** the fact that the third and fourth hemispherical portions (23, 24) being respectively convex and concave, the two faces (41, 42) of the screwing head (32) and of the portion of hemispherical shape that come into contact with each other are respectively convex and concave.

## Patentansprüche

1. System für die Wirbelsäulen-Osteosynthese, das wenigstens eine Pedikelschraube (1) mit einem Kopf (5), eine Klammer (2), erste Mittel (10) zum Befestigen eines ersten Endes (4) der Klammer (2) am Kopf (5) der Pedikelschraube, eine Platte (6) und zweite Mittel (20) zum Befestigen der Platte (6) am zweiten Ende (7) der Klammer (2) umfasst, wobei die ersten Mittel (10) zum Befestigen des ersten Endes (4) der Klammer am Kopf (5) der Pedikelschraube umfassen:
einen ersten Teil (11) mit Halbkugelform, der mit dem Kopf (5) der Pedikelschraube (1) fest verbunden ist,
einen zweiten Teil (12) mit Halbkugelform, der mit dem ersten Ende (4) der Klammer (2) fest verbunden ist, wobei dieser zweite Halbkugelteil (12) zu dem ersten Halbkugelteil (11) komplementär ist, und
erste Befestigungsmittel (13) zum Befestigen des ersten und des zweiten Halbkugelteils (11, 12) aneinander, wenn sie ineinander zusammenwirken, indem sie dasselbe erste Krümmungszentrum (14) besitzen, und
wobei die zweiten Mittel (20) zum Befestigen der Platte (6) am zweiten Ende (7) der Klammer (2) umfassen:
einen dritten Teil (23) mit Halbkugelform, der mit der Platte (6) fest verbunden ist,
einen vierten Teil (24) mit Halbkugelform, der mit dem zweiten Ende (7) der Klammer (2) fest verbunden ist, wobei dieser vierte Halbkugelteil (24) zu dem dritten Halbkugelteil (23) komplementär ist, und
zweite Befestigungsmittel (25) zum Befestigen des dritten und des vierten Halbkugelteils (23, 24) aneinander, wenn sie ineinander zusammenwirken, indem sie dasselbe zweite Krümmungszentrum (26) haben.

2. System nach Anspruch 1, **dadurch gekennzeichnet, dass** die ersten und die zweiten Befestigungsmittel (13, 25) eine Fixierschraube (30), die aus einem Gewindestift (31) und aus einem Aufschraubkopf (32) gebildet ist, und Mittel zum Anbringen der Fixierschraube (30) in Zusammenwirkung mit den zwei Halbkugelteilen (11-12, 23-24), derart, dass einer dieser zwei Teile zwischen dem anderen Teil und dem Aufschraubkopf (32) sandwichartig angeordnet wird, umfassen.

3. System nach Anspruch 2, **dadurch gekennzeichnet, dass** die Mittel zum Anbringen der Fixierschraube (30) in Zusammenwirkung mit den zwei Halbkugelteilen, um einen dieser zwei Teile zwischen dem anderen Teil und dem Aufschraubkopf (32) sandwichartig anzuordnen, **dadurch** gebildet sind, dass der Gewindestift (31) mit jenem Halbkugelteil fest verbunden ist, der den anderen sandwichartig aufnimmt, und durch diesen anderen Halbkugelteil durch ein Loch (40) verläuft, dessen Querschnitt größer als jener des Gewindestifts (31) ist, wobei der Aufschraubkopf (32) auf den Gewindestift (31) geschraubt wird, wobei die zwei Seiten (41, 42) des Aufschraubkopfes (32) und des Halbkugelteils, die miteinander in Kontakt gelangen, komplementäre Halbkugelformen haben und Krümmungszentren besitzen, die mit dem Krümmungszentrum (14, 26) der ineinander zusammenwirkenden Halbkugelteile im Wesentlichen zusammenfallen.

4. System nach Anspruch 3, **dadurch gekennzeichnet, dass** dann, wenn der erste und der zweite Halbkugelteil (11, 12) konvex bzw. konkav sind, die zwei Seiten (41, 42) des Aufschraubkopfes (32) und des Halbkugelteils, die miteinander in Kontakt gelangen, konkav bzw. konvex sind.

5. System nach Anspruch 2, **dadurch gekennzeichnet, dass** die Mittel zum Anbringen der Fixierschraube (30) in Zusammenwirkung mit den zwei Halbkugelteilen, um eines dieser zwei Teile sandwichartig zwischen dem anderen Teil und dem Aufschraubkopf (32) anzuordnen, **dadurch** gebildet sind, dass der Gewindestift (31) in jenen Halbkugelteil geschraubt wird, der den anderen sandwichartig aufnimmt, und durch diesen anderen Teil durch ein Loch (40) verläuft, dessen Querschnitt größer als jener des Gewindestifts (31) ist, wobei der Aufschraubkopf (32) mit dem Gewindestift (31) fest verbunden ist, wobei die zwei Seiten (41, 42) des Aufschraubkopfes (32) und des Halbkugelteils, die miteinander in Kontakt gelangen, komplementäre Halbkugelformen haben und Krümmungszentren besitzen, die mit dem Krümmungszentrum (14, 26) der ineinander zusammenwirkenden Halbkugelteile im Wesentlichen zusammenfallen.

6. System nach Anspruch 5, **dadurch gekennzeichnet, dass** dann, wenn der dritte und der vierte Halbkugelteil (23, 24) konvex bzw. konkav sind, die zwei Seiten (41, 42) des Aufschraubkopfes (32) und des Halbkugelteils, die miteinander in Kontakt gelangen, konvex bzw. konkav sind.
